# EUROPEAN PATENT APPLICATION

(11) **EP 2 865 406 A1**
(43) Date of publication of application: **29.04.2015**
(21) Application number: 14174582.8
(22) Date of filing: 26.06.2014
(51) Int. Cl.: A61M 25/09

(54) **Guide wire**

(30) Priority: 25.10.2013 JP 2013222631
(71) Applicant: ASAHI INTECC CO., LTD., Nagoya-shi, Aichi 463-0024 (JP)
(72) Inventor: Miyata, Naohiko c/o ASAHI INTECC CO., LTD., Nagoya-shi, Aichi 463-0024 (JP); Ikeno, Jun c/o ASAHI INTECC CO., LTD., Nagoya-shi, Aichi 463-0024 (JP); Sato, Kazuo c/o ASAHI INTECC CO., LTD., Nagoya-shi, Aichi 463-0024 (JP)
(74) Representative: Winter, Brandl, Fürniss, Hübner, Röss, Kaiser, Polte - Partnerschaft mbB

(57) **Abstract**

[Problem] Provided is a guide wire which is excellent in the flexibility of a distal portion thereof, the followability for a blood vessel and the like and the transmutability of delicate informations such as resistance produced when the distal portion makes contact with a wall surface of a blood vessel and the like to slide, resistance produced when the distal portion bumps against a stenosed section and the like.

[Solution] A guide wire comprising; a core shaft and a coil body 1 covering a distal portion of the core shaft, the coil body 1 being formed by helically winding a stranded wire having two or more elemental wires interwound together, and the stranded wire being hollow and having a first void at the center thereof.

## Description

### TECHNICAL FIELD

The present invention relates to a medical guide wire used as a guide when inserting a catheter into a blood vessel, a gastrointestinal tract, a ureter and the like; inserting an indwelling device; and the like.

### BACKGROUND ART

When inserting a catheter into a blood vessel, a gastrointestinal tract, a ureter and the like for treatment and examination; inserting an indwelling device; and the like, a guide wire is used in order to guide them.

In general, flexibility is required for a guide wire so that an inner wall of a blood vessel, a gastrointestinal tract and the like may not be damaged. In particular, excellent flexibility is required for a distal portion thereof. For example, Patent Literature 1 discloses a guide wire in which flexibility is improved by providing a shaft formed with a stranded wire. Further, Patent Literature 2 discloses a guide wire in which a shaft comprises a core wire and two or more fine wires interwound around the core wire, but does not comprise the core wire at a distal portion thereof, thereby achieving higher flexibility at the distal portion as compared with that at the body portion.

Meanwhile, in recent years, the range of intended uses for a guide wire has been increasingly expanding. In particular, since blood vessels are intricately bended, a guide wire used for blood vessels often requires more excellent flexibility and followability for blood vessels at a distal portion thereof. A guide wire comprising a shaft formed with a stranded wire as disclosed in the above Patent Literatures 1 and 2 can show excellent flexibility. However, different problems may arise when fine wires in the stranded wire are made thinner in order to further improve flexibility. For example, the followability for blood vessels may decrease due to decreased rigidity and rotary torque transmutability of a guide wire. Or, the following delicate informations at a distal portion which can otherwise be sensed through a grip by an operator operating a guide wire from the outside of the body may not be well transmitted: resistance produced when the distal portion of the guide wire inside the body makes contact with a wall surface of a blood vessel and the like, and resistance produced when the distal portion of the guide wire bumps against a stenosed section.

### Citation List

### Patent Literatures

Patent Literature 1: Japanese Patent Laid-Open No. H10-323395
Patent Literature 2: Japanese Patent Laid-Open No. 2001-293092

### SUMMARY OF THE INVENTION

### Technical Problem

An object of the present invention is to provide a guide wire which is excellent in the flexibility at a distal portion thereof, in the followability for a complex structure such as a blood vessel, and in the transmittability of delicate informations such as resistance produced when the distal portion makes contact with a wall surface of a blood vessel and the like to slide, and resistance procured when the distal portion bumps against a stenosed section.

### Solution to Problem

A guide wire according to the first embodiment of the present invention comprises a core shaft and a coil body covering a distal portion of the core shaft, the coil body being formed by helically winding a stranded wire having two or more elemental wires interwound together, and the stranded wire being hollow and having a first void at the center thereof.

Further, a guide wire according to the second embodiment of the present invention comprises a core shaft, a first coil body covering a distal portion of the core shaft, a second coil body covering the distal portion of the core shaft and connected to a proximal end of the first coil body, and a third coil body arranged within the first coil body and the second coil body and covering the distal portion of the core shaft, the first coil body being formed by helically winding a stranded wire having two or more elemental wires interwound together, and the stranded wire being hollow and having a first void at the center thereof, wherein an interface between the first coil body and the second coil body is joined only with the third coil body.

### Advantageous Effects of the Invention

The guide wire according to the first embodiment of the present invention comprises a core shaft and a coil body, the coil body being formed by helically winding a stranded wire having two or more elemental wires interwound together, and the stranded wire being hollow and having a first void, but not a core wire at the center thereof. Since the coil body formed by helically winding a stranded wire having a first void at the center is provided as described above, a distal portion of the guide wire has very much excellent flexibility, is capable of moving flexibly, and shows excellent followability for a blood vessel and the like. Furthermore, it has excellent transmutability of delicate informations such as vibration due to resistance produced at the distal portion. Moreover, since the unevenness on a surface of the coil body is small, the guide wire can smoothly slide along a wall surface of a blood vessel, a gastrointestinal tract, a ureter and the like.

The guide wire according to the second embodiment of the present invention comprises a core shaft, a first coil body covering a distal portion of the core shaft, a second coil body connected to a proximal end of the first coil body and covering the distal portion of the core shaft, and a third coil body arranged within the first coil body and the second coil body and covering the distal portion of the core shaft, the first coil body being formed by helically winding a stranded wire having two or more elemental wires interwound together, and the stranded wire being hollow and having a first void but not a core wire at the center thereof. Further, an interface between the first coil body and the second coil body is joined only with the third coil body. Since the coil body formed by helically winding a stranded wire having a first void at the center is provided as described above, the distal portion of the guide wire has very much excellent flexibility, is capable of moving flexibly, and shows excellent followability for a blood vessel and the like. Furthermore, it has excellent transmutability of delicate informations such as vibration due to resistance produced at the distal portion. Moreover, since the unevenness on a surface of the coil body is small, the guide wire can smoothly slide along a wall surface of a blood vessel, a gastrointestinal tract, a ureter and the like. Furthermore, since multiple coil bodies such as the first coil body, the second coil body and the third coil body are provided and an interface joining only these coil bodies is provided, flexibility, rotary torque transmutability and the like can be easily adjusted by the configuration of the coil bodies.

### BRIEF DESCRIPTION OF DRAWINGS

Fig. 1 shows a front view schematically illustrating a surface of the coil body used in the first aspect,
Fig. 2 shows a front view schematically illustrating the stranded wire used in the first aspect,
Fig. 3 shows a cross-sectional view schematically illustrating the stranded wire used in the first aspect,
Fig. 4 shows a cross-sectional view schematically illustrating another example of the stranded wire used in the first aspect,
Fig. 5 shows a cross-sectional view schematically illustrating the stranded wire shown in figure 4 in another state,
Fig. 6 shows a cross-sectional view schematically illustrating the stranded wire shown in figure 4 in yet another state,
Fig. 7 shows a cross-sectional view schematically illustrating yet another example of the stranded wire used in the first aspect,
Fig. 8 shows a cross-sectional view schematically illustrating the first aspect of the guide wire according to the present invention,
Fig. 9 shows an enlarged view of the region A in Fig. 8,
Fig. 10 shows an enlarged view of another example of the region A in Fig. 8,
Fig. 11 shows a cross-sectional view schematically illustrating the second aspect of the guide wire according to the present invention, and
Fig. 12 shows a front view schematically illustrating a surface of the coil body in Comparative Example.

### DESCRIPTION OF EMBODIMENTS

Embodiments of the present invention will be described below with reference to the drawings, but the present invention shall not be limited to these.

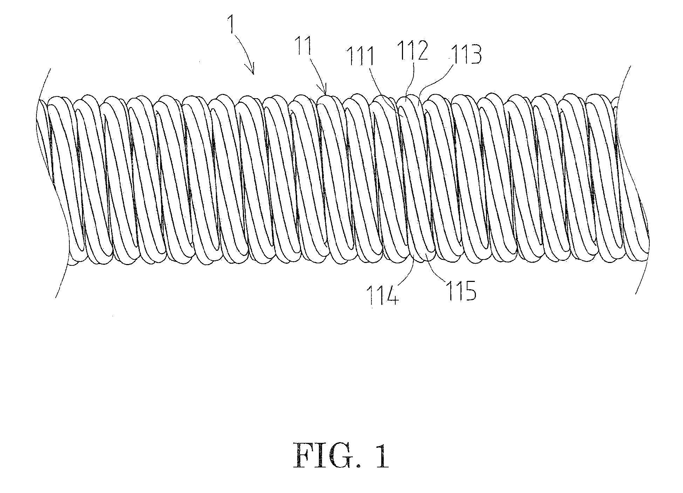

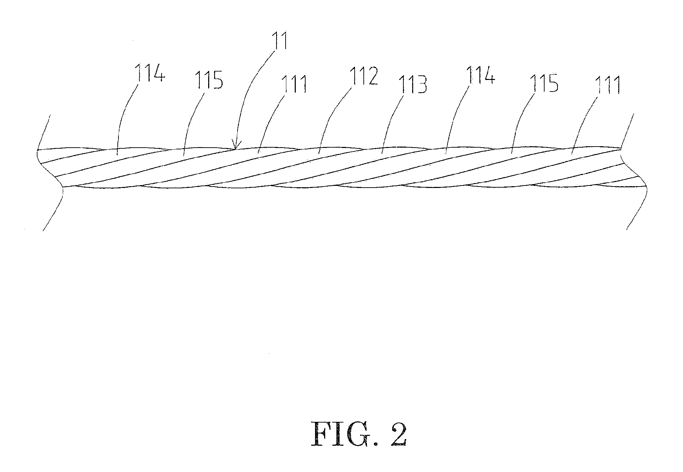

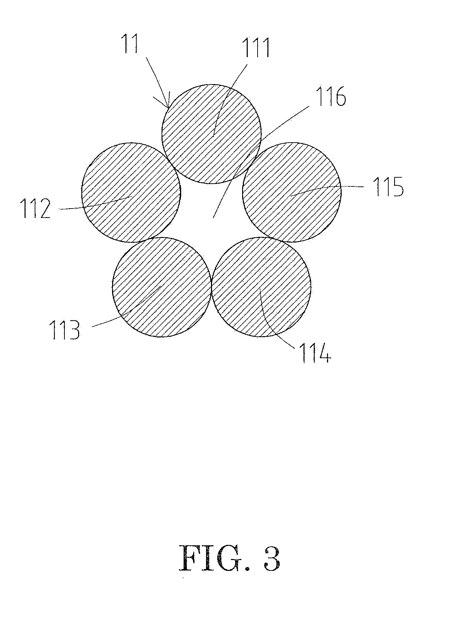

Fig. 1 shows a front view schematically illustrating a surface of the coil body used in the first aspect of the guide wire of the present invention. Fig. 2 shows a front view schematically illustrating a state of the stranded wire before wound into a coil body. Fig. 3 shows a cross-sectional view of the stranded wire shown in Fig. 2.

A coil body 1 used in the first aspect of the guide wire according to the present invention is formed by helically winding a stranded wire 11 having two or more elemental wires interwound together.

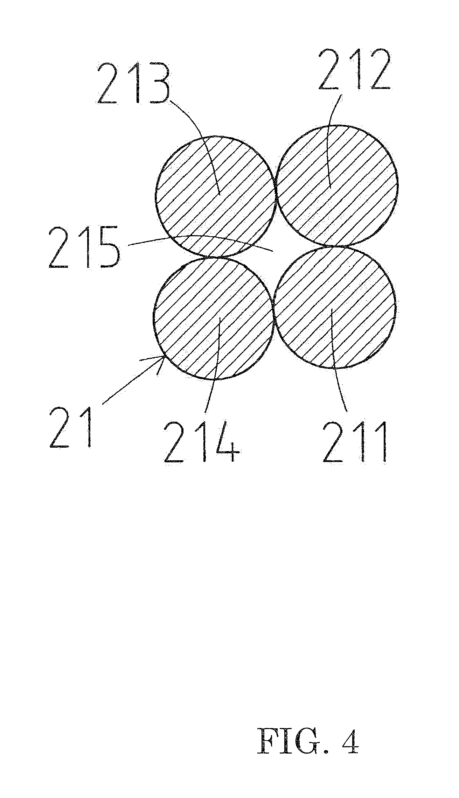

The stranded wire 11 comprises two or more elemental wires interwound together. The lower limit of the number of elemental wires in the stranded wire 11 is preferably 3 while the upper limit is preferably 8, more preferably 6. In Figs. 1, 2 and 3, the stranded wire 11 comprises 5 elemental wires 111, 112, 113, 114 and 115, and a first void 116, but not a core wire at its center. Further, the number of elemental wires in the stranded wire is most preferably 4 as shown in Fig. 4. In Fig. 4, the stranded wire 21 comprises 4 elemental wires 211, 212, 213 and 214, and a first void 215, but not a core wire at its center.

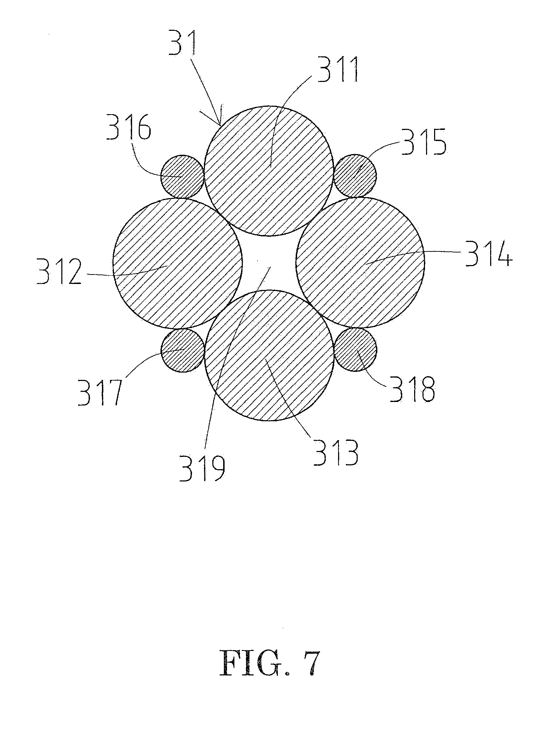

Further, the stranded wire may comprise elemental wires having different diameters as shown in Fig. 7. In Fig. 7, the stranded wire 31 comprises first elemental wires 311, 312, 313 and 314 having the substantially same diameter, and further comprises second elemental wires 315, 316, 317 and 318 having smaller diameters than those of the first elemental wires between the first elemental wires, and comprises a first void 319 but not a core wire at its center. In the stranded wire 31 as described above, a gap between the first elemental wires 311, 312, 313 and 314 is occupied by the second elemental wires 315, 316, 317 and 318. Therefore, even in a case where after the stranded wire is helically wound into a coil body, a brazing material, an adhesive and the like are used to join the coil body, and even in a case where the coil body is covered with a coating material, the brazing material, the adhesive, the coating material and the like do not easily enter into the first void 319 at the center of the stranded wire 31 through the gap between the first elemental wires 311, 312, 313 and 314.

As shown in Fig. 7, in a case where a stranded wire comprises elemental wires having different diameters, the lower limit of the number of the first elemental wires is preferably 3 while the upper limit is preferably 8, more preferably is 6 and most preferably 4. The number of the second elemental wires is preferably selected, depending on the number of the gaps between the first elemental wires.

Elemental wires used in the above number ranges will ensure the formation of the first voids 116, 215 and 319 at the center of the resulting stranded wires 11, 21 and 31, and confer the shape stability on the stranded wires 11, 21 and 31. Moreover, in a case where the stranded wires 11, 21 and 31, which are to be helically wound into a coil body, are used to form coil bodies, the unevenness produced on a surface of the coil body will be small. Therefore, a distal portion of a guide wire obtained using the above coil body will smoothly slide along a vessel wall of a blood vessel and the like.

The stranded wires 11, 21 and 31 are formed by interwinding two or more elemental wires together. There is no particular limitation for the manner of interwinding as long as the resulting stranded wires 11, 21 and 31 are hollow and have the first voids 116, 215 and 319 at the center. However, the stranded wires 11, 21 and 31 are preferably formed by helically interwinding two or more elemental wires together in the same pitch and direction because the shape stability, shape uniformity and surface smoothness of the resulting stranded wires are improved. Further, at elemental wires adjacent each other, one elemental wire is preferably interwound over another elemental wire in the direction of interwinding.

Details will be described with referring to Fig. 2. As shown in Fig. 2, the stranded wire 11 comprises 5 elemental wires 111, 112, 113, 114 and 115, and the 5 elemental wires are helically interwound together in the same direction. For example, in the case of Fig. 2, they are helically interwound in the counterclockwise direction toward the left side in the figure. More specifically, at a certain point, the 5 elemental wires are circularly arranged in this order of the elemental wires 111, 112, 113, 114 and 115 in the counterclockwise direction toward the left side in the figure. They are interwound together such that any one elemental wire is arranged over an adjacent elemental wire in the counterclockwise direction (the direction of interwinding). That is, at a certain point, the elemental wire 115 is located in the counterclockwise direction of the elemental wire 114, and the elemental wire 114 is interwound over the elemental wire 115, and then the elemental wire 113 is interwound over the elemental wire 114. Further, the elemental wire 112 is interwound over the elemental wire 113, and the elemental wire 111 is interwound over the elemental wire 112, and the elemental wire 115 is interwound over the elemental wire 111 again. By repeating the above procedure, the stranded wire 11 can be obtained which is hollow and has the first void 116 but not a core wire at the center.

Although a case in which 5 elemental wires are interwound together is described above, a similar strategy can be applied to a case where 4 elemental wires are interwound together as shown in Fig. 4, a case where 3 or not less than 6 elemental wires are interwound together, and even a case where elemental wires having different diameters are interwound together as shown in Fig. 7.

Materials for elemental wires include stainless steel, superelastic alloys such as a Ni-Ti alloy and the like. The two or more elemental wires may be made of the same material, or elemental wires made of different materials may be used in combination.

Examples of a shape of a cross-section of an elemental wire include circular, elliptical and polygonal such as square and rectangle, but it is preferably circular because excellent interwinding properties, excellent shape stability of the resulting stranded wire and excellent smoothness of a surface of the resulting stranded wire can be obtained.

In a case where a shape of a cross-section of an elemental wire is circular as shown in Figs. 3 and 4, the diameters of two or more elemental wires are preferred to be substantially the same. In this case, the upper limit of the diameter of an elemental wire is preferably 0.05 mm, and the lower limit is preferably 0.01 mm. Further, the stranded wire may comprise elemental wires having different diameters as shown in Fig. 7. In that case, the upper limit of the diameter of the first elemental wires 311 to 314 is preferably 0.05 mm, and the lower limit is preferably 0.01 mm.

The above stranded wires 11, 21 and 31 do not have core wires but have the first voids 116, 215 and 319 at the center. Therefore, the relative positional relationship among two or more elemental wires in the stranded wires 11, 21 and 31 may change depending on the force applied to the stranded wires 11, 21 and 31. For example, when the stranded wires 11, 21 and 31 are helically wound, the stranded wires 11, 21 and 31 which are straight or curved will be transformed into a helical form. As this transform undergoes, the relative positional relationship among the elemental wires may change to some extent. As a result of this, the stranded wires 11, 21 and 31 will be more easily helically wound, and in addition, unevenness will be less likely formed on a surface of the resulting helically wound coil body. Further, even after preparing a guide wire using a coil body obtained by helically winding the stranded wires 11, 21 and 31, the coil body may undergo deformation to some extent when advancing in a winding blood vessel and the like. Even in that case, the relative positional relationship among two or more elemental wires in the stranded wires 11, 21 and 31 will change to some extent as the coil body undergoes deformation to control the repelling force against the deformation of the coil body. This can confer excellent flexibility and followability for a blood vessel and the like on the coil body.

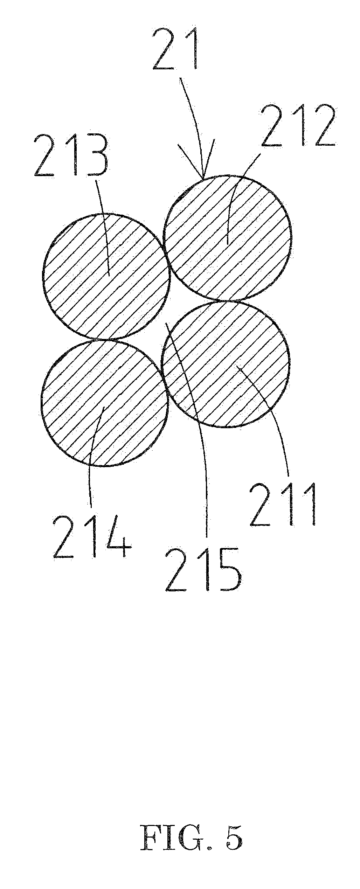

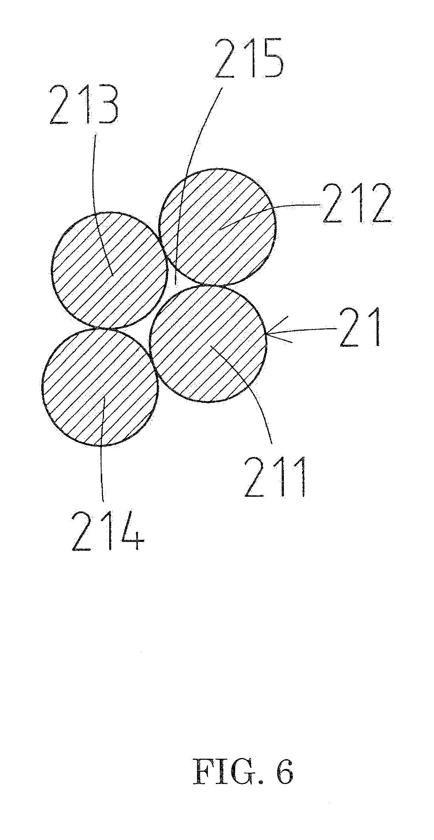

Figs. 4, 5 and 6 show schematic cross-sectional views to illustrate the relative positional relationship of the stranded wire 21 comprising 4 elemental wires 211, 212, 213 and 214. When the stranded wire 21 in the state as shown in Fig. 4 undergoes deformation to some extent, the relative positional relationship among the elemental wires 211, 212, 213 and 214 will change to some extent as shown in Fig. 5, depending on the deformation of the stranded wire 21. When the stranded wire 21 further undergoes deformation, the relative positional relationship among the elemental wires 211, 212, 213 and 214 will further change as shown in Fig. 6. In this way, the relative positional relationship among the elemental wires 211, 212, 213 and 214 changes to some extent depending on the deformation of the stranded wire 21, resulting in reduced repelling force against deformation.

Meanwhile, since the elemental wires 211, 212, 213 and 214 are interwound together, even in a case where the relative positional relationship among the elemental wires 211, 212, 213 and 214 changes to some extent, the elemental wires 211, 212, 213 and 214 will not fall apart by unwinding, and the shapes of the stranded wire 21 and the coil body 2 formed by helically winding the stranded wire 21 will not be destroyed.

As described above, the stranded wires 11, 21 and 31 having the first voids 116, 215 and 319 at the center show excellent flexibility for deformation. Therefore, coil bodies formed by helically winding the stranded wires 11, 21 and 31 as described above show less unevenness on their surfaces for easy sliding, and further have excellent flexibility as well as excellent followability for a blood vessel and the like.

The above stranded wire is to be helically wound into a coil body.

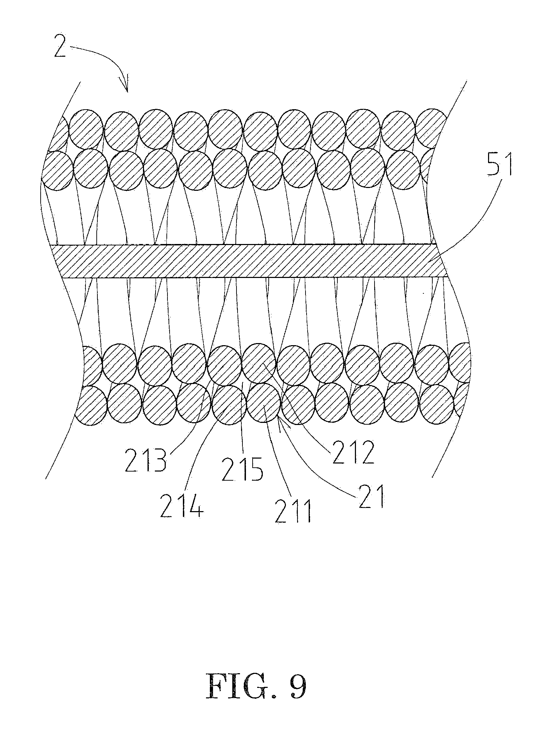

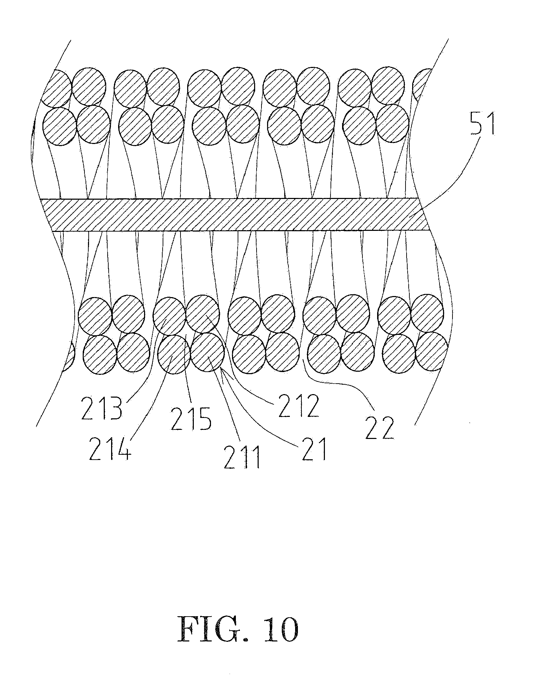

As shown in Figs. 1 and 9, the stranded wires 11 and 21 are wound so that rounds of the stranded wires 11 and 21 to be wound make tight contact each other. As shown in Fig. 10 described below, a configuration is also preferred in which rounds of the stranded wire 21 helically wound do not make tight contact each other, but has a second void 22 between the rounds of the stranded wire 21. By providing the second void 22 between the rounds of the stranded wire 21 in the coil body 2, the flexibility of a distal portion of the resulting guide wire and the followability for a blood vessel and the like can be further improved. Moreover, the transmutability, to the hand of an operator, of delicate vibration produced when the distal portion of the guide wire contacts with a wall surface of a blood vessel and the like and a stenosed section is further improved.

Further, there is no particular limitation for the relationship between the direction of interwinding the elemental wires in the stranded wires 11 and 21 together, and the direction of winding the stranded wires 11 and 21 in the coil bodies 1 and 2. In a case where the coil bodies 1 and 2 are formed by helically winding the stranded wires 11 and 21 interwound in the counterclockwise direction, the coil bodies 1 and 2 may be of either clockwise or counterclockwise helical. Further, in a case where the coil bodies 1 and 2 are formed by helically winding the stranded wires 11 and 21 interwound in the clockwise direction, the coil bodies 1 and 2 may also be of either clockwise or counterclockwise helical.

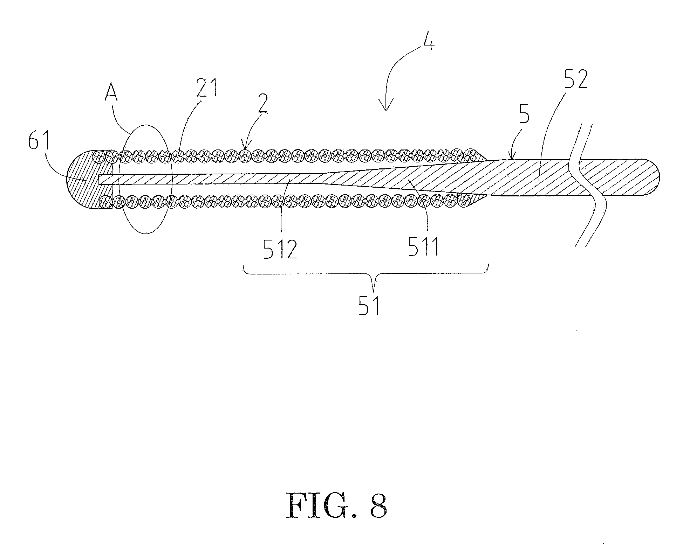

Fig. 8 shows a cross-sectional view schematically illustrating the first aspect of the guide wire according to the present invention. In the figure, the left side corresponds to a distal side (tip side) to be inserted into the body, while the right side corresponds to a proximal side (base end side) to be operated by an operator.

A guide wire 4 comprises a core shaft 5 and the coil body 2 covering a distal portion 51 of the core shaft 5. The coil body 2 is formed by helically winding the above stranded wire 21.

The guide wire 4 is a flexible elongated article, and the length and diameter thereof can be suitably selected depending on the range of intended uses. The upper limit of the length is generally 4000 mm, and preferably 2500 mm. The lower limit of the length is generally 500 mm, and preferably 1000 mm. In the first aspect, the length of the guide wire 4 is about 1800 mm. Further, in general, the maximum diameter of a cross section of the guide wire 4 is from 0.05 mm to 0.5 mm.

The above core shaft 5 is a flexible elongated member, and has a length and maximum diameter depending on the length and maximum diameter of the guide wire 4. The shape of a cross-section of the core shaft 5 may be any of circular, elliptical and polygonal such as square and rectangle, but is generally and preferably circular.

The core shaft 5 comprises a distal portion 51 located in a distal side to be inserted into the body upon treatment, examination and the like, and a body portion 52 located in a proximal side of the distal portion 51.

In a case where the distal portion 51 is tapered in diameter toward the distal side to improve flexibility toward the distal side, and in a case where the guide wire 4 is intended for treating a blood vessel in the heart, the distal portion 51 is generally provided in a region from the distal end of the guide wire 4 up to 400 mm axially toward the proximal side. For example, the distal portion 51 is gradually tapered toward the distal side by providing a tapered portion 511 tapered in diameter toward the distal side. At the distal side of the tapered portion 511, a reduced diameter portion 512 extending in the axial direction with the substantially same diameter is arranged. The distal end of the reduced diameter portion 512 is joined with the distal end of the coil body 2 through a joining means. There is no particular limitation for the joining means as long as it has a smooth shape so that the distal end of the guide wire 4 does not damage a wall surface of a blood vessel and the like, and a conventionally known joining method may be used. In general, as shown in Fig. 8, they are joined through a distal end tip 61 having a smooth apical surface.

Note that a tapered portion in a core shaft may not be limited to one as described above, but two or more may be formed.

The distal end tip 61 has a smooth apical surface comprising a curved surface such as a hemispherical surface so that a blood vessel and the like are not damaged. There is no particular limitation for a forming method thereof as long as the distal end of the core shaft 5 can be joined with the distal end of the coil body 2. For example, the distal end tip 61 is preferably formed by assembling components such as the core shaft 5 and the coil body 2, and then providing a brazing material to the distal end to join the distal end of the core shaft 5 with the distal end of the coil body 2 by brazing.

In the guide wire 4 as shown in Fig. 8, the distal end of the distal portion 51 of the core shaft 5 is joined with the distal end tip 61, but it may be configured such that the distal end of the distal portion 51 is spaced from and not joined with the distal end tip 61. In that case, the core shaft 5 is preferably connected to the distal end tip 61 via a safe wire and the like. Further, even in a case where the distal end of the distal portion 51 of the core shaft 5 is joined with the distal end tip 61, the core shaft 5 may be further connected to the distal end tip 61 via a safe wire and the like.

The body portion 52 of the core shaft 5 corresponds to a portion extending from a proximal end of the distal portion 51 in the axial direction with the substantially same diameter, which is a portion other than the distal portion 51 of the core shaft 5. The distal side of the body portion 52 is to be inserted into the body following the distal portion 51 upon treatment and examination while a proximal side remains exposed outside the body.

For materials for the core shaft 5, conventionally known materials can be used. They include stainless steel, superelastic alloys such as a Ni-Ti alloy, a piano wire and the like. Among others, stainless steel is preferred. The entire core shaft 5 may be made of the same material, or different materials may be used in part.

The coil body 2 formed by helically winding the above stranded wire 21 comprising 4 elemental wires and having the first void 215 at the center can be used as a coil body, but the coil body 1 comprising the above stranded wires 11 may also be used. The coil body 2 is joined with the distal end tip 61 at the distal end, and a proximal end thereof is joined with the core shaft 5 near the proximal end of the distal portion 51 of the core shaft 5 by a conventionally known joining method such as brazing, soldering and adhesion with an adhesive.

As shown in Fig. 8, the coil body 2 is provided so that the outer periphery of the distal portion 51 of the core shaft 5 is covered. The coil body 2 may be entirely or partly formed with the stranded wire 21. In a case where a part of the coil body 2 is formed with the stranded wire 21, at least a region of the distal end of the coil body 2, preferably at least a region up to 20 mm from the distal end, more preferably at least a region up to 30 mm from the distal end is preferably formed with the stranded wire 21. Note that in a case where a part of the coil body 2 is formed with the stranded wire 21, a conventionally known coil body can be appropriately used for other portions.

Fig. 9 shows an enlarged view of the region A in Fig. 8. The coil body 2 is arranged at the outer periphery of the distal portion 51 of the core shaft 5. The coil body 2 is formed by helically winding the stranded wire 21. The stranded wire 21 is formed by interwinding 4 elemental wires 211, 212, 213 and 214 together, and has the first void 215 at the center.

The stranded wire 21 is helically wound tightly so that adjacent rounds of the stranded wire 21 make contact each other, and a configuration as shown in Fig. 10 is also preferred in which the adjacent rounds of the stranded wire 21 do not make contact, and the second void 22 is formed between the rounds of the stranded wire 21.

There is no particular limitation for methods of manufacturing the above guide wire 4, but they include, for example, the following methods.

First, the coil body 2 is made by interwinding two or more elemental wires 211, 212, 213 and 214 together to form the stranded wire 21, and helically winding the resulting stranded wire 21 over a core material having a desired diameter, and then withdrawing the core material. Then, the core shaft 5 fabricated into a desired shape is inserted into the coil body 2. The coil body 2 is aligned with the core shaft 5, and then the proximal end of the coil body 2 is joined with the outer periphery of the core shaft 5 by a conventionally known joining method such as brazing, soldering, and adhesion with an adhesive, and then the distal end of the coil body 2 is joined with the distal end of the core shaft 5 with a brazing material and the like. When joining the distal end of the coil body 2 with the distal end of the core shaft 5 using a brazing material and the like, the distal end tip 61 is formed.

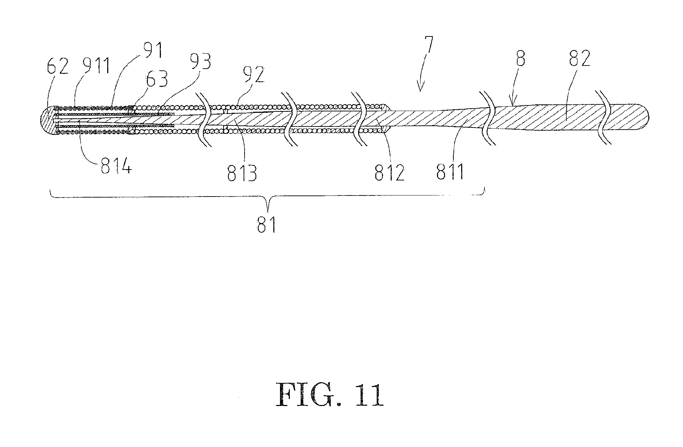

Fig. 11 shows a cross-sectional view schematically illustrating the second aspect of the guide wire according to the present invention. In the figure, the left side corresponds to a distal side (tip side) to be inserted into the body while the right side corresponds to a proximal side (base end side) to be operated by an operator.

A guide wire 7, which is intended for treating a blood vessel in the heart and the like, comprises a core shaft 8, a first coil body 91 covering a distal portion 81 of the core shaft 8, a second coil body 92 covering the distal portion 81 of the core shaft 8 and connected to a proximal end of the first coil body 91, and a third coil body 93 arranged within the first coil body 91 and the second coil body 92 and covering the distal portion 81 of the core shaft 8.

The core shaft 8, which is similar to the core shaft 5 according to the first aspect, comprises the distal portion 81 located at a distal side to be inserted into the body upon treatment, examination and the like, and a body portion 82 located at a proximal side of the distal portion 81.

In the guide wire 7 as shown in Fig. 11, the distal portion 81 has, in this order from the proximal side, a first tapered portion 811 tapered in diameter toward the distal side, a first reduced diameter portion 812 extending in the axial direction with the substantially same diameter, a second tapered portion 813 tapered in diameter toward the distal side and a second reduced diameter portion 814 extending in the axial direction with the substantially same diameter and connected to a distal end tip 62. However, it is not limited to this configuration as in the case of the above core shaft 5.

Further, the distal end of the distal portion 81 is joined with the distal end tip 62, but a configuration may also be possible in which the distal end of the distal portion 81 is spaced from the distal end tip 62, and the distal portion 81 is not joined with the distal end tip 62 as in the case of the first aspect. In that case, the core shaft 8 is preferably connected to the distal end tip 62 via a safe wire and the like.

The first coil body 91 covers the outer periphery of the distal side including the distal end in the distal portions 81 of the core shaft 8, and is formed by helically winding the stranded wire 911 having two or more elemental wires interwound together.

The first coil body 91 is joined with the distal end tip 62 at the distal end, and the proximal end is joined with the distal end of the second coil body 92 and an intermediate portion of the third coil body 93 at an interface portion 63.
The distal end tip 62 has a similar configuration as the above distal end tip 61 according to the first aspect. The first coil body 91 is joined with the distal end tip 62 in a similar joining fashion as the coil body 2 joined with the distal end tip 61 in the first aspect. A conventionally known joining method such as brazing, soldering and adhesion with an adhesive may be used for joining at the interface portion 63.

For the stranded wire 911, one similar to the stranded wire 21 according to the first aspect may be used. In short, the first coil body 91 has a similar configuration as the coil body 2 according to the first aspect.

In Fig. 11, a case in which the stranded wire 911 comprises 4 elemental wires is shown, but the present invention is not limited to this as in the first aspect.

The first coil body 91 covers the outer periphery of the distal side including the distal end in the distal portions 81 of the core shaft 8, and preferably covers a region preferably at least up to 20 mm from the distal end of the guide wire 7 toward the proximal side in the axial direction, and more preferably at least up to 30 mm.

The above second coil body 92 is provided continuously at the proximal end of the first coil body 91. The outer diameter of the second coil body 92 is substantially the same as that of the proximal end of the first coil body 91. The second coil body 92 covers the outer periphery of a portion in which the first coil body 91 does not cover in the distal portion 81 of the core shaft 8. The distal end of the second coil body 92 is joined with the proximal end of the first coil body 91 and the intermediate portion of the third coil body 93 via the interface portion 63. The proximal end of the second coil body 92 is joined near the proximal end of the distal portion 81 of the core shaft 8 by a conventionally known joining method such as brazing, soldering and adhesion with an adhesive.

The second coil body 92 may be formed with a coil body similar to the first coil body 91. However, it is preferred to be appropriately selected for use from conventionally known other coil bodies. Conventionally known other coil bodies include a coil body formed by helically winding an elemental wire, and a coil body formed by helically winding a stranded wire comprising two or more elemental wires and having a core wire.

Materials for an elemental wire used for the second coil body 92 include stainless steel, superelastic alloys such as a Ni-Ti alloy and the like.

The third coil body 93 is arranged within the first coil body 91 and the second coil body 92, and covers the outer periphery of the distal side including the distal end in the distal portions 81 of the core shaft 8. The distal end of the third coil body 93 is joined with the distal end tip 62, and the proximal end is joined with the core shaft 8 at a position between the interface portion 63 and the proximal end of the second coil body 92 by a conventionally known joining method such as brazing, soldering and adhesion with an adhesive. The third coil body 93 is joined with the distal end tip 62 in a similar joining fashion as the coil body 2 joined with the distal end tip 61 in the first aspect.

The third coil body 93 is arranged within the first coil body 91 and the second coil body 92, and is one which has been conventionally used as a so-called an inner coil. The third coil body 93 may be formed with a coil body similar to the first coil body 91. However, it is preferred to be appropriately selected for use from other conventionally known inner coils. Conventionally known inner coils include a coil body formed by helically winding a stranded wire comprising two or more elemental wires and having a core wire, a coil body formed by helically winding an elemental wire and the like.

Materials for an elemental wire used for the third coil body 93 include stainless steel, superelastic alloys such as a Ni-Ti alloy and the like.

The interface portion 63 is joined with the proximal end of the first coil body 91, with the distal end of the second coil body 92 and with the intermediate portion of the third coil body 93, but not with the core shaft 8. As described above, since only the first to third coil bodies 91, 92 and 93 are joined via the interface portion 63, the flexibility of the first to third coil bodies 91, 92 and 93 is not impaired by the interface portion 63. In addition, since the first to third coil bodies 91, 92 and 93 are joined together, the guide wire 7 has excellent rigidity, rotary torque transmutability and the like.

Since for the guide wire 7 according to the second aspect, the outer periphery of the distal portion 81 including the distal end of the core shaft 8 is covered with the first coil body 91, the distal portion of the guide wire 7 is very much excellent in flexibility and followability for a blood vessel and the like. Further it has excellent transmutability, to the hand of an operator, of delicate vibration produced at the first coil body 91 when the distal portion of the guide wire 7 makes contact with a wall surface of a blood vessel and the like and a stenosed section. Further, since the second coil body 92 is connected to the proximal end of the first coil body 91, the flexibility, followability for a blood vessel and the like, rotary torque followability, rigidity and the like can be easily appropriately adjusted by appropriately selecting the second coil body 92 depending on a range of intended uses and desired physical properties. Further, since the third coil body 93 is arranged within the first coil body 91 and the second coil body 92, the flexibility of the distal portion can be easily improved while maintaining excellent rotary torque followability by appropriately selecting the third coil body 93 depending on a range of intended uses and desired physical properties. Furthermore, since the interface portion 63 joins with only the proximal end of the first coil body 91, the distal end of the second coil body 92 and the intermediate portion of the third coil body 93, but not with the core shaft 8, the flexibility, rotary torque followability and the like is not impaired by the interface portion 63.

There is no particular limitation for methods of manufacturing the above guide wire 7, but they include, for example, the following methods.

First, the first coil body 91 is made by interwinding two or more elemental wires to form the stranded wire 911 having a first void at the center, helically winding the resulting stranded wire 911 over a core material having a desired diameter, and then withdrawing the core material. Further, by a similar method, the second coil body 92 and the third coil body 93 are made by separately winding an elemental wire or a stranded wire in a helical fashion. Next, the core shaft 8 fabricated into a desired shape is inserted into the third coil body 93, and the third coil body 93 is aligned with the core shaft 8, and then the proximal end of the third coil body 93 are joined with the core shaft 8 by a conventionally known joining method such as brazing, soldering, and adhesion with an adhesive. Then, the third coil body 93 and the core shaft 8 are inserted into the second coil body 92, and the second coil body 92 is aligned with the core shaft 8, and then the proximal end of the second coil body 92 are joined with the core shaft 8 by a conventionally known joining method such as brazing, soldering, and adhesion with an adhesive. Further, the third coil body 93 and the core shaft 8 are inserted into the first coil body 91, and the distal end of the second coil body 92 is aligned with the proximal end of the first coil body 91. Then the proximal end of the first coil body 91, the distal end of the second coil body 92 and the intermediate portion of the third coil body 93 are joined by a conventionally known joining method such as brazing, soldering, and adhesion with an adhesive to form the interface portion 63. Finally, the distal end of the first coil body 91, the distal end of the third coil body 93 and the distal end of the core shaft 8 are joined using a brazing material. At this time, the distal end tip 62 comprising the brazing material is formed.

Next, a method of using the guide wire 4 according to the first aspect and the guide wire 7 according to the second aspect for treatment and examination will be described with reference to a case in which they are used for a stenosed section formed in a coronary artery of the heart.

The guide wires 4 and 7 are inserted into an artery from the femoral region and the like, and allowed to pass through the aortic arch to advance toward the stenosed section formed in the coronary artery, which is a target site for treatment. At this time, an operator such as a doctor applies pushing force and torque to the guide wires 4 and 7. The guide wires 4 and 7 are excellent in the flexibility of the distal portion and the followability for a blood vessel and the like. Therefore, even in the case of complicatedly bended blood vessels, they are allowed to smoothly follow a blood vessel without damaging a wall of the blood vessel to smoothly advance towards the stenosed section. Further, the guide wire 7 has the first coil body 91, the second coil body 92 and the third coil body 93. Therefore, insertion properties when applying such pushing force, rotary torque transmutability when applying torque, and the like can be easily adjusted by appropriately selecting the second coil body 92 and the third coil body 93. Furthermore, the guide wires 4 and 7 are excellent in the transmutability of delicate informations such as resistance procured when the distal portion makes contact with a wall surface of a blood vessel and the like to slide, resistance produced when the distal portion bumps against a stenosed section and the like. Therefore, an operator can advance the guide wire while sensing resistance applied to the distal portion.

After the guide wires 4 and 7 reach a target site for treatment, for example, a therapeutic catheter such as a balloon catheter and a treatment device-introducing catheter is inserted into the body along the guide wires 4 and 7 to perform treatment such as dilation of a stenosed section.

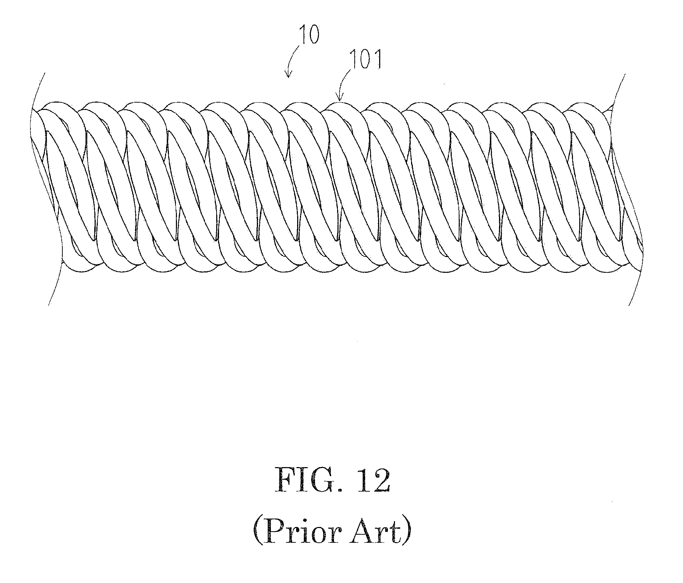

Fig. 12 shows a front view schematically illustrating a surface of a coil body formed with a stranded wire having no void at the center, that is, a conventionally known stranded wire having a core wire, for comparison.

A coil body 10 is formed by helically winding a stranded wire 101.

The stranded wire 101 is formed by helically interwinding 4 circular elemental wires comprising stainless steel and having a circular cross section of about 0.03 mm in diameter around a circular core wire (an elemental wire) comprising stainless steel and having a circular cross section of about 0.015 mm in diameter. The stranded wire is interwound as in the above embodiments 1 and 2 except that the core wire is used.

A surface of the coil body 10 formed with the stranded wire 101 having a core wire at the center, which is as shown in Fig. 12, shows more unevenness on the surface as compared with that of the coil body used for the present invention as shown in Fig. 1.

### Description of Reference Numerals

1, 2: Coil body
11, 21, 31, 911: Stranded wire
116, 215, 319: First void
211, 212, 213, 214, 111, 112, 113, 114, 115: Elemental wire
22: Second void
311, 312, 313, 314: First elemental wire
315, 316, 317, 318: Second elemental wire
4, 7: Guide wire
5, 8: Core shaft
51, 81: Distal portion
511: Tapered portion
512: Reduced diameter portion
52, 82: Body portion
61, 62: Distal end tip
63: Interface portion
811: First tapered portion
812: First reduced diameter portion
813: Second tapered portion
814: Second reduced diameter portion
91: First coil body
92: Second coil body
93: Third coil body

## Claims

1. A guide wire (4), comprising:
a core shaft (5); and
a coil body (1, 2) covering a distal portion (51) of the core shaft (5);
the coil body (1, 2) being formed by helically winding a stranded wire (11, 21, 31) having two or more elemental wires (111, 112, 113, 114, 115,211,212,213,214, 311, 312, 313, 314) interwound together, and the stranded wire (11, 21, 31) being hollow and having a first void (116, 215, 319) in the center thereof.

2. A guide wire (4) according to claim 1, comprising a second void (22) between rounds of the stranded wire (21) wound helically.

3. A guide wire (7) comprising:
a core shaft (8);
a first coil body (91) covering a distal portion (81) of the core shaft (8);
a second coil body (92) covering the distal portion (81) of the core shaft (8), and connected to a proximal end of the first coil body (91); and
a third coil body (93) arranged within the first coil body (91) and the second coil body (92), and covering the distal portion (81) of the core shaft (8),
the first coil body (91) being formed by helically winding a stranded wire (21, 911) having two or more elemental wires (211, 212, 213, 214) interwound together, and
the stranded wire (21, 911) being hollow and having a first void (215) at the center thereof,
wherein an interface portion (63) between the first coil body (91) and the second coil body (92) is joined only with the third coil body (93).

4. A guide wire (7) according to claim 3, comprising a second void (22) between rounds of the stranded wire (21, 911) wound helically.

5. A guide wire (4, 7) according to any one of claims 1 to 4, wherein the number of the two or more elemental wires in the stranded wire (21, 911) is 4.

6. A guide wire (4, 7) according to any one of claims 1 to 4, wherein the stranded wire (31) comprises first elemental wires (311, 312, 313, 314), and second elemental wires (315, 316, 317, 318) having diameters smaller than those of the first elemental wires, and the second elemental wires (315, 316, 317, 318) are arranged between the first elemental wires (311, 312, 313, 314).
